(19) **Europäisches Patentamt European Patent Office Office européen des brevets**

(11) **EP 4 504 229 B1**

(12) **FASCICULE DE BREVET EUROPEEN**

(45) Date de publication et mention de la délivrance du brevet:
**04.03.2026 Bulletin 2026/10**

(21) Numéro de dépôt: **23722618.8**

(22) Date de dépôt: **07.04.2023**

(51) Classification Internationale des Brevets (IPC):
*A61K 36/899* (2006.01)   *A61K 36/38* (2006.01)
*A61P 17/10* (2006.01)   *A61K 9/00* (2006.01)

(52) Classification Coopérative des Brevets (CPC):
(C-Sets disponibles)
**A61P 17/10; A61K 9/0014; A61K 36/38; A61K 36/899**   (Cont.)

(86) Numéro de dépôt international:
**PCT/FR2023/050509**

(87) Numéro de publication internationale:
**WO 2023/194696 (12.10.2023 Gazette 2023/41)**

(54) **ASSOCIATION D'UN EXTRAIT DE GARCINIA MANGOSTANA ET D'UN JUS D'AVENA SATIVA FRAICHE POUR LUTTER CONTRE L'INFLAMMATION INDUITE PAR C. ACNES**

KOMBINATION AUS EINEM GARCINIA MANGOSTANA EXTRAKT UND FRISCHEM AVENA SATIVA SAFT ZUR BEHANDLUNG VON C. ACNES VERURSACHTEN ENTZÜNDUNGEN

ASSOCIATION OF AN EXTRACT OF GARCINIA MANGOSTANA AND JUICE OF FRESH AVENA SATIVA FOR TREATING C. ACNES INDUCED INFLAMMATION

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité: **07.04.2022 FR 2203205**

(43) Date de publication de la demande:
**12.02.2025 Bulletin 2025/07**

(73) Titulaire: **Pierre Fabre Dermo-Cosmétique 81500 Lavaur (FR)**

(72) Inventeurs:
• **LETI, Mathieu**
  **81106 CASTRES (FR)**
• **GARIDOU, Lucile**
  **81106 CASTRES (FR)**
• **DUPLAN, Hélène**
  **81106 CASTRES (FR)**
• **MAITRE, Martine**
  **81106 CASTRES CEDEX (FR)**

(74) Mandataire: **Regimbeau**
  **20, rue de Chazelles**
  **75847 Paris Cedex 17 (FR)**

(56) Documents cités:
EP-B1- 3 046 429   WO-A2-2007/002666
FR-A1- 2 938 439   US-A1- 2006 292 255

• CHOMNAWANG ET AL: "Effect of Garcinia mangostana on inflammation caused by Propionibacterium acnes", FITOTERAPIA, IDB HOLDING, MILAN, IT, vol. 78, no. 6, 23 August 2007 (2007-08-23), pages 401 - 408, XP022208876, ISSN: 0367-326X, DOI: 10.1016/ J.FITOTE.2007.02.019

(52) Classification Coopérative des Brevets (CPC):
(Cont.)

C-Sets
**A61K 36/38, A61K 2300/00;**
**A61K 36/899, A61K 2300/00**

**Description**

**DOMAINE TECHNIQUE DE L'INVENTION**

La présente invention concerne une nouvelle association comprenant un extrait de Garcinia mangostana L. et un jus d'*Avena sativa* fraiche ainsi que des compositions comprenant cette association, en particulier pour leur utilisation dans le domaine de l'acné, et notamment dans le traitement de l'inflammation induite par *C. acnes*.

**ETAT DE LA TECHNIQUE**

L'acné est une affection cutanée chronique inflammatoire de l'unité pilo-sébacée entrainant une formation de comédons, touchant le visage, la région scapulaire, les bras et les régions intertrigineuses. Elle est la principale cause des dermatoses les plus fréquentes. Il est important de ne pas banaliser cette affection et de la traiter correctement car elle peut avoir des conséquences psychosociales invalidantes notamment du fait de la formation de cicatrices.

L'acné est une pathologie multifactorielle. En effet, divers facteurs de risques peuvent conduire au développement de l'acné ou l'accentuer. Ces facteurs concernent l'imprégnation hormonale et le patrimoine génétique mais aussi des facteurs externes environnementaux, comme la pollution et les UVs. Enfin, le mode de vie peut également influencer l'apparition ou l'amplification de l'acné, à savoir l'alimentation et le stress. Il existe plusieurs formes d'acné. Elle peut d'abord se traduire par l'apparition de simples comédons (points noirs et points blancs). On parle alors d'acné rétentionnelle. Mais ces lésions rétentionnelles peuvent aussi évoluer vers des lésions inflammatoires plus sévères : les papules, pustules et nodules. On parle dans ce cas d'acné inflammatoire. Cependant, il est désormais établi que l'inflammation est présente à tous les stades de l'acné même les plus précoces, c'est-à-dire les stades infracliniques : on parle de micro-inflammation. Classiquement ces deux formes coexistent : il s'agit de l'acné mixte, concernant près de 60% des acnéiques.

L'acné commune ou acné vulgaire, également appelée acné polymorphe juvénile, est la plus courante et comprend quatre stades :

- Le stade 1 correspond à l'acné comédonnienne ou rétentionnelle et est caractérisé par un grand nombre de comédons ouverts et/ou fermés et de microkystes ;
- Le stade 2, ou acné papulo-pustuleuse, est de gravité légère à modérée et est caractérisé par la présence de comédons ouverts et/ou fermés, de microkystes, mais également de papules et pustules rouges. Il affecte principalement le visage et laisse quelques cicatrices ;
- Le stade 3, ou acné papulo-comédonnienne, est plus grave et s'étend au dos, au thorax et aux épaules. Il est accompagné d'un grand nombre de cicatrices ;
- Le stade 4, ou acné nodulo-kystique, présente des nodules et également des pustules pourpres volumineux et douloureux. Il est accompagné de nombreuses cicatrices.

Sous la forme la plus légère, l'acné concerne presque chaque être humain. Sa fréquence est maximale à l'âge de la puberté, mais elle peut se manifester pour la première fois dès l'âge de 7 à 9 ans et jusqu'à des âges dépassant 40 ans. Il est fréquent de souffrir d'acné encore après 25 ans. L'acné touche en outre aussi bien les hommes que les femmes. Au cours de la puberté, sous l'influence des sécrétions hormonales et en particulier des androgènes, mais aussi associé à différents facteurs externes, il est observé une surproduction de sébum appelée hyperséborrhée. Chez des sujets prédisposés à l'acné, cet environnement est propice au développement de la bactérie clé de l'acné, *Cutibacterium acnes* (*C. acnes*) (anciennement appelée *Propionibacterium acnes*). Cette bactérie métabolise les triglycérides cutanés en acide gras irritants *via* des lipases qui agressent la paroi du follicule et du derme environnant, produit également divers enzymes et chimioattractants des cellules phagocytaires de l'immunité, et stimule la production de cytokines pro-inflammatoires par différents types de cellules (sébocytes, kératinocytes, monocytes, notamment) qui aggravent l'inflammation. Cette bactérie joue un rôle pivot dans l'acné, notamment en stimulant la réponse inflammatoire locale (Dagnelie et al., Journal of the European Academy of Dermatology 2019, 33(12), 2340-2348). On a longtemps pensé que la lutte contre cette espèce bactérienne était prioritaire dans le traitement de l'acné. L'usage de divers antimicrobiens topiques sont aujourd'hui encore largement utilisés (peroxyde de benzoyle, érythromycine, triclosan). Mais depuis peu, les chercheurs ont compris que le but n'est pas d'éradiquer *C. acnes* qui est une bactérie commensale, nécessaire à l'homéostasie tissulaire, mais de rétablir un équilibre. En effet, on pense aujourd'hui que l'acné serait directement liée à l'appauvrissement du microbiote cutané (dysbiose), beaucoup moins diversifié chez les personnes souffrant d'acné que chez les individus sains. Par ailleurs, l'altération du microbiote cutané entraine des modifications de pH cutané et déclenche l'inflammation.

De même, une antibiothérapie systémique plus ou moins prolongée était parfois associée au traitement selon la gravité de l'affection (tétracyclines, doxycycline). Les dermatologues vont aujourd'hui plus se tourner vers des anti-inflammatoires locaux, séborégulateurs.

On a constaté des échecs fréquents à tous ces traitements, dus souvent à une forte proportion de souches résistantes de *C. acnes.* FR 2 938 439 décrit l'utilisation d'un extrait des parties aériennes d'Avena sativa pour le traitement de l'acné.

Ainsi, il existe toujours un besoin de fournir des traitements plus efficaces de l'acné ne présentant pas d'effets indésirables chez le patient.

## RESUME DE L'INVENTION

La présente invention concerne une association comprenant un extrait de Garcinia mangostana et un jus d'Avena sativa fraiche ainsi qu'une composition cosmétique ou dermatologique comprenant une telle association et au moins un excipient cosmétiquement ou dermatologiquement acceptable.

L'invention concerne également une association comprenant un extrait de Garcinia mangostana et un jus d'Avena sativa fraiche ainsi qu'une composition cosmétique ou dermatologique comprenant une telle association et au moins un excipient cosmétiquement ou dermatologiquement acceptable pour leur utilisation dans le traitement de l'inflammation induite par *C. acnes* ou dans le traitement d'une peau acnéique.

D'autres aspects de l'invention sont tels que décrits ci-après et dans les revendications.

## DESCRIPTION DETAILLEE DE L'INVENTION

La présente invention a pour objet de répondre aux besoins exprimés. En effet, les inventeurs ont mis en évidence, de façon tout à fait inattendue, que l'association d'un extrait de Garcinia mangostana L. et d'un jus d'*Avena sativa* fraiche avaient la capacité de réduire la production de cytokines inflammatoires, l'inflammation étant stimulée en présence de *C. acnes.* En effet, l'association d'un extrait de Garcinia mangostana L. et d'un jus d'*Avena sativa* fraiche présente l'avantage d'agir de façon synergique sur la cascade immuno-inflammatoire médiée par *C. acnes* et d'être ainsi utile dans le traitement de l'acné.

La présente invention a donc pour objet une association comprenant un extrait de *Garcinia mangostana* et un jus d'*Avena sativa* fraiche, ainsi qu'une composition cosmétique ou dermatologique comprenant une telle combinaison et leurs utilisations dans le traitement de l'inflammation induite par *C. acnes* ou le traitement d'une peau acnéique.

L'extrait de Garcinia mangostana et le jus d'*Avena sativa* fraiche utile dans le cadre de la présente invention peuvent être tels que décrits ci-après.

Le terme « environ » telle qu'employé ci-après signifie que la valeur concernée peut être inférieure ou supérieure de 10%, notamment de 5%, en particulier de 1%, par rapport à la valeur indiquée.

## Extrait de Garcinia mangostana

*Garcinia mangostana* L. est un arbre tropical originaire d'Asie du Sud-Est, aujourd'hui cultivé dans de nombreux pays tropicaux pour son fruit comestible, le mangoustan, également appelé « fruit des Dieux » ou « reine des fruits ».

*Garcinia mangostana* L. est un arbre dioïque pouvant atteindre 20 mètres de hauteur à l'état sauvage. Ses feuilles sont lisses, luisantes, coriaces, elliptiques à elliptiques-oblongues, mesurant 14 à 25 cm sur 5 à 10 cm. Leur base est cunéiforme à subarrondie, l'apex est courtement acuminé. Les fleurs mâles, rares, sont groupées 2 à 9 à l'extrémité de brindilles, tandis que les fleurs femelles, un peu plus grandes que les fleurs mâles, sont solitaires ou par paires. Le fruit est constitué d'un épais péricarpe de couleur rose à violacé très sombre à maturité, renfermant une chair blanche comestible et enveloppant 4 à 5 graines.

Le mangoustan est connu depuis des siècles dans la médecine traditionnelle asiatique pour ses activités antioxydante, anti-inflammatoire et antibactérienne. Le péricarpe notamment est utilisé en cas d'infection cutanée, de diarrhées, de douleurs abdominales, d'infection urinaire, ou de contusions. Il peut également être utilisé en cas de fièvre (Ovalle-Magallanes et al., Food Chem Toxicol 109 : 102-122, 2017). On observe également en occident un fort engouement pour le mangoustan depuis une dizaine d'années. De nombreux jus de fruits à base de chair et de péricarpe, compléments alimentaires et produits topiques contenant du mangoustan sont commercialisés et de récentes études cliniques montrent leur intérêt dans diverses indications telles que la perte de poids (Udani et al., Nutr J, 8 : 48, 2009) et la parodontite (Mahendra et al., J Investig Clin Dent, 8(4), 2017). On trouve également des extraits de mangoustan comme actifs dans plusieurs références cosmétiques pour leurs propriétés anti-âge ou amincissante.

Le péricarpe de mangoustan est une source importante de xanthones dont les principales sont l'$\alpha$-mangostine et la $\gamma$-mangostine (Ovalle-magallanes et al., 2017). Le péricarpe de mangoustan contient par ailleurs des tanins, des anthocyanes et des sucres. Des extraits de fruits, et plus particulièrement de péricarpe de mangoustan plus ou moins enrichis en xanthones ont fait l'objet de nombreuses études pharmacologiques, à la fois *in vitro* et *in vivo.* Les principales activités mises en évidence sont des propriétés antitumorales, notamment au niveau de la prostate, du poumon, du sein et du côlon, anti-inflammatoires, antioxydantes, anti-diabétiques, anti-hyperlipidémiques et antibactériennes.

Dans la présente invention, la plante Garcinia mangostana L. est désignée de manière abrégée par le terme « Garcinia mangostana ».

Par « extrait de Garcinia mangostana », on entend désigner le produit d'extraction de tout ou partie de la plante Garcinia mangostana.

Par « produit d'extraction », on entend le produit obtenu après extraction d'une partie de la plante, avec un solvant, appelé solvant d'extraction, c'est-à-dire un produit présent dans le solvant d'extraction qui peut ensuite être éventuellement sous une forme concentrée ou sèche après évaporation partielle ou totale du solvant d'extraction. Il peut s'agir d'un extrait sec.

Par « extrait sec », on entend au sens de la présente invention, un extrait dépourvu de solvant d'extraction ou de support, ou en contenant uniquement à l'état de trace non significative.

Un tel extrait sec contient ainsi uniquement de la matière issue de Garcinia mangostana. Il peut contenir également des traces non significatives de solvant d'extraction. Cette définition s'applique en particulier lorsque l'on définit la teneur en poids d'alpha-mangostine par rapport au poids de l'extrait sec.

Typiquement, l'extrait de Garcinia mangostana est obtenu à partir d'une ou plusieurs parties aériennes de la plante Garcinia mangostana telles que le fruit, le péricarpe du fruit, la pulpe du fruit, les graines, les feuilles, les tiges et/ou l'écorce.

Dans certains modes de réalisation, l'extrait est obtenu à partir des fruits et/ou des péricarpes du fruit de Garcinia mangostana. De façon avantageuse, l'extrait est obtenu à partir des péricarpes du fruit de Garcinia mangostana.

Dans certains modes de réalisation, l'extrait est obtenu à partir d'une culture de cellules de Garcinia mangostana.

Dans certains modes de réalisation, l'extrait de Garcinia mangostana, en particulier l'extrait des péricarpes du fruit de Garcinia mangostana, est un extrait hydro-alcoolique, en particulier un extrait hydro-éthanolique.

L'extrait de Garcinia mangostana, en particulier l'extrait des péricarpes du fruit de Garcinia mangostana, est susceptible d'être obtenu par un procédé tel que décrit ci-après.

L'extrait de Garcinia mangostana utile dans le cadre de la présente invention peut ainsi être préparé par un procédé de préparation comprenant une étape d'extraction de tout ou partie de plante Garcinia mangostana par un solvant hydrophile à apolaire, de préférence un solvant moyennement polaire à apolaire. La plante ou partie de plante Garcinia mangostana utile à la préparation de l'extrait de Garcinia mangostana peut être fraiche ou sèche, entière, coupée, ou broyée puis soumise à une étape d'extraction.

Par « solvant hydrophile », on entend un solvant choisi dans le groupe constitué notamment de l'eau, de l'eau subcritique, des alcools miscibles à l'eau comme par exemple l'éthanol, des glycols en C3 à C5, du glycérol, de l'acétone, et des mélanges de ceux-ci.

Par « solvant apolaire », il faut entendre au sens de la présente invention, un solvant choisi par exemple parmi l'heptane, l'hexane, le limonène, les hydrocarbures halogénés (chloroforme, dichlorométhane), le $CO_2$ supercritique, un mélange $CO_2$ supercritique et éthanol.

Par « solvant moyennement polaire », on entend au sens de la présente invention, un solvant choisi dans le groupe constitué notamment des alcools en C1 à C5, des glycols comme le propylène glycol, le butylène glycol, le butanediol, ou le pentylène glycol, de glycérol, d'acétone, d'esters d'alkyles tels qu'acétate d'éthyle, acétate d'isopropyle, de solvants miscibles à l'eau (un mélange hydro-alcoolique ou un mélange acétone/eau par exemple). Figurent également dans ce groupe des solvants alternatifs de type hydrotropes (molécules amphiphiles solubles dans l'eau et qui, à partir d'une concentration suffisante, peuvent extraire des composés moyennement polaires tels que décrits dans la caractérisation de l'extrait).

Dans certains modes de réalisation de l'invention, le solvant d'extraction est choisi parmi l'acétate d'éthyle, l'acétate d'isopropyle, un mélange $CO_2$ supercritique et éthanol, un glycol en C3 à C5, un alcool de C1 à C5, un mélange alcool/eau, une solution hydrotropique ou un mélange de ceux-ci. Avantageusement, il s'agit d'un mélange éthanol/eau ou d'une solution hydrotropique. De façon plus avantageuse, il s'agit d'un mélange éthanol/eau. De façon encore plus avantageuse, ce mélange éthanol/eau est caractérisé par une proportion éthanol/eau de 9 : 1 à 7 : 3 (v/v). Et de façon encore plus avantageuse, le solvant moyennement polaire est un mélange éthanol/eau dans la proportion 9 : 1 (v/v).

L'extraction peut être réalisée sous agitation ou de façon statique, à reflux, à température ambiante, ou à une température comprise entre la température ambiante et le reflux. Elle peut être assistée par ultra-sons, par micro-ondes, par flash détente ou par extrusion, dans un ratio poids de plante/volume de solvant pouvant varier de 1/3 à 1/30, pendant une durée de 1 minute à 48 heures. L'extraction peut être renouvelée 2 à 3 fois.

Le marc peut être ensuite séparé de l'extrait par centrifugation ou filtration afin de récupérer une phase liquide limpide exempte de particules. La phase liquide représentant l'extrait peut être plus ou moins concentrée pouvant aller jusqu'à l'obtention d'un extrait sec. Ainsi, le procédé de préparation de l'extrait de Garcinia mangostana peut comprendre une étape de concentration.

Dans certains modes de réalisation, un support peut être rajouté lors de l'étape de concentration de façon à obtenir un extrait de Garcinia mangostana contenant 1 à 75% en poids d'extrait sec.

Le support peut être de la maltodextrine, du lactose, de la silice, de la glycérine, un glycol, une huile végétale, un hydrotrope, un mélange eau/solubilisant ou eau/tensioactif, ou tout autre support cosmétologiquement acceptable et solubilisant l'extrait, préférentiellement d'origine biosourcée comme par exemple des glycols biosourcés (1,2-pentanediol ; 1,3-butanediol ; 1,3-propanediol...), des acides gras estérifiés et également les hydrotropes comme par exemple les alkyls glycosides (Sepiclear, Apyclean, APXC4...).

Dans certains modes de réalisation, le procédé de préparation de l'extrait de Garcinia mangostana comprend une étape de décoloration. Ainsi, l'extrait de Garcinia mangostana peut être décoloré, par exemple sur charbon actif. De façon avantageuse, l'extrait de Garcinia mangostana n'est pas décoloré.

Jus d'Avena sativa fraiche

L'avoine ou Avena sativa L. est une plante annuelle de la famille des poacées pouvant atteindre 1 m 50 de haut. A la germination, la jeune plante est gazonnante puis donne plusieurs tiges.

La tige ou chaume est creuse, de quelques millimètres de diamètre, interrompue de place en place, à l'endroit où s'insèrent les feuilles, par des diaphragmes pleins appelés nœuds. Les entre-nœuds, d'abord très courts à la base de la tige, deviennent de plus en plus longs.

Le fruit ou grain de l'avoine est reconnu comme une matière première médicinale par voie orale comme laxatif pour son effet de lest mais également dans le cas de cholestérol ou de diabète de type 2 pour sa teneur en $\beta$-glucanes. Par voie topique, le fruit d'avoine est utilisé principalement sous forme de farine dont une qualité, appelée extrait colloïdal d'avoine, a une monographie à la Pharmacopée Américaine USP 22, 1990. Cet extrait colloïdal présente des propriétés émollientes et adoucissantes, et est défini comme étant la poudre résultant du broyage et autres traitements du grain entier, cette qualité correspond à une farine d'avoine. A partir de cette même partie de l'avoine, on peut extraire également de l'huile utilisée en cosmétologie et des protéines. Ces dernières, insolubles ne sont pas utilisées directement mais après hydrolyse enzymatique ou chimique. On réalise un hydrolysat plus ou moins poussé qui permet d'obtenir, soit des peptides d'avoine de poids moléculaires variables, soit des acides aminés selon la force de l'hydrolyse. Les protéines d'avoine hydrolysées ont été examinées pour leurs propriétés dans les domaines cosmétique et dermatologique. Ainsi, il a été démontré des propriétés sur le cheveu comme la faculté qu'ont ces peptides à former un film sur la tige capillaire, de pénétrer dans la cuticule et ainsi par l'effet gainant résultant d'apporter un effet conditionneur.

En dehors des grains, l'avoine est utilisée comme plante fourragère. Coupée jeune, elle donne un fourrage vert très estimé. La paille est quant à elle donnée aux chevaux, vaches et moutons, mais n'est pas utilisée en alimentation humaine. En médecine traditionnelle, la paille d'avoine sert à la préparation de bains apaisants des douleurs rhumatismales, de la sciatique et des affections hépatiques. En inde, des décoctions d'avoine ordinaire servaient au sevrage de toxicomanes sous l'emprise d'opium. Un extrait alcoolique préparé à partir de plantes fraiches a été utilisé dans le sevrage tabagique avec des résultats statistiquement significatifs.

L'avoine « herbe » fait l'objet d'une monographie EMEA (Référence EMEA/HMPC/202966/2007) dans laquelle est mentionnée l'utilisation soit des parties aériennes récoltées avant floraison et séchées, soit d'un extrait liquide (1 : 5, éthanol 45% v/v) préparé à partir des parties aériennes fraiches de la plante récoltée pendant la période de floraison. L'utilisation traditionnelle de ces parties aériennes est décrite en cas de léger stress mental et pour favoriser l'endormissement. D'après la bibliographie, les parties aériennes d'avoine sont composées de :

- Flavonoïdes :

  ∘ C-glycosyl-flavones de type apigenine (vitexine, isovitexine...) ou luteoline (orientine, isoorientine, isoscoparine ...),
  ∘ Flavones de type tricine, O-glycosylés,
  ∘ Flavolignanes (salcolines A et B),

- Saponines stéroïdes bidesmosidiques : Avenacosides A et B (aglycone : nuatigenin),
- Protéines,
- Autres : composés phénoliques (Avénanthramides, acides hydroxycinnamiques...), stérols, cérébrosides...

Dans la présente invention, la plante *Avena sativa* L. ainsi que sa variété Rhéa, renommée Rhealba est désignée de manière abrégée par le terme *Avena sativa.*

Par « *Avena sativa* fraiche », on entend au sens de la présente invention les parties aériennes de l'avoine, à l'exclusion des graines seules, utilisées fraiches ou (dé)congelées, composées de 30 à 90% d'eau, de préférence 40 à 90% d'eau, de préférence encore de 40 à 80% d'eau, en poids. On comprend ainsi que les parties aériennes de l'avoine utilisées pour produire le jus n'ont pas subi de dessication et sont traitées rapidement après leur récolte pour limiter la perte en eau. Elles peuvent être congelées pour être traitées plus tard.

De manière préférée, les parties aériennes de l'avoine sont les parties aériennes de plantules d'avoine.

Le jus d'*Avena sativa* fraiche utile dans le cadre de l'invention est ainsi un jus de parties aériennes d'avoine. Plus particulièrement le jus d'*Avena sativa* fraiche est un jus de parties aériennes de plantules d'avoine.

Les plantules représentent la partie de la plante récoltée avant épiaison au stade mi-montaison, soit environ 2 mois après la mise en culture, c'est-à-dire avant l'apparition des fleurs et des fruits (ou grains). A ce stade la plante est dépourvue de protéines de pollen et des grains.

Par « parties aériennes », on entend ici les tiges et les feuilles et les fleurs et dans le cas de plantules seulement les tiges et les feuilles. Ces parties aériennes ne comprennent pas les fruits (appelés aussi grains) et, dans le cas de plantules elles ne comprennent pas non plus les fleurs. La plante n'est pas séchée mais peut être congelée afin de garder ses caractéristiques de fraicheur.

Par « jus d'*Avena sativa* fraiche », on entend au sens de la présente invention l'eau d'*Avina sativa,* contenant les molécules d'intérêt, sans aucune dénaturation. Les plantes fraiches, c'est-à-dire non déshydratées, en particulier les plantules, et plus particulièrement les parties aériennes de plantules, sont soumises à un traitement thermomécanique consistant à extruder l'*Avina sativa* fraiche dans un extrudeur, associé à un traitement thermique permettant d'inactiver les enzymes endogènes et de conserver les molécules de composés d'intérêt sous leur forme native, en l'absence de solvant, suivi d'une opération de récupération du jus.

Il est ainsi clair qu'un « jus » est distinct d'un « extrait ». Dans le cadre de la préparation d'un « extrait », les molécules d'intérêt sont retirées de la plante au moyen d'un solvant qui peut être de l'eau, un solvant organique ou leur mélange. L'obtention d'un jus ne nécessite le recours à aucun solvant. Le jus correspond donc à la fraction liquide contenue dans la plante et extraite de la plante, dans le cas présent par traitement thermomécanique.

Le jus d'avoine fraiche (INCI : *Avena sativa* (OAT) Flower/Leaf/Stem Juice) correspond ainsi à l'eau naturellement présente dans les parties aériennes de l'avoine, et plus particulièrement les parties aériennes de plantules d'avoine.

Lors d'un pressage de plantes fraiches selon des techniques traditionnelles, la paroi végétale freine parfois la récupération de certains composés d'intérêt. De plus, les enzymes natives présentes dans la plante sont facilement libérées et peuvent commencer à modifier les composés extraits dans le jus et à les dénaturer : hydrolyses, oxydations, déglycosylations, etc.

L'adaptation d'une technique d'extrusion largement utilisée en alimentaire pour cuire et expanser des matières, à des fins d'extraction a ainsi permis de récupérer un jus natif de la plante fraiche sans utilisation de solvant ni d'eau dans lequel les molécules actives n'ont pas été modifiées ou altérées par les enzymes endogènes.

En effet, l'extrusion permet de déstructurer complètement les parois végétales de la plante et le traitement thermique très court (quelques secondes) permet de neutraliser les enzymes sans détériorer les molécules d'intérêt présentes.

Cette technique est décrite en détail dans la demande de brevet WO 2015/040135.

Selon le procédé appliqué pour obtenir le jus d'avoine fraiche, particulièrement le jus de parties aériennes de plantules d'avoine fraiche, par « extrusion », on entend un traitement thermomécanique consistant à extruder la plante fraiche, particulièrement les parties aériennes de plantules d'avoine fraiche, dans un extrudeur, de préférence un extrudeur bi-vis, associé à un traitement thermique.

Ainsi pour obtenir un jus d'avoine fraiche, particulièrement le jus de parties aériennes de plantules d'avoine fraiche, l'extrusion se caractérise par le passage de la plante fraiche, particulièrement les parties aériennes de plantules d'avoine fraiche, dans un extrudeur bi-vis composé de :

- une zone d'introduction des plantes fraiches, particulièrement des parties aériennes de plantules d'avoine fraiche : Trémie d'alimentation
- le corps principal de l'extrudeuse est constitué d'un ou plusieurs fourreaux dans lesquels tournent les vis sans fin (corotatives ou contrarotatives), ou segments de vis.

De préférence, il s'agit de plusieurs fourreaux successifs adjacents. De préférence, il s'agit de deux vis sans fin corotatives. Le profil des vis peut varier selon la forme du filet des vis, par ex. : trapézoïdal, conjugué, simple ou double...) et du pas de vis. Chacune de ces vis peut également présenter différents tronçons (ou segments) qui peuvent éventuellement différemment les uns des autres, par la forme fu filet et/ou par le pas de vis. Eventuellement, certains des tronçons constitutifs de ces vis peuvent également correspondre à des éléments malaxeurs monolobes, ou trilobés ;

- au moins un fourreau filtrant qui : intervient le cas échéant pour la séparation solide/liquide ; comprend en outre un moyen de filtration, tel qu'une grille et ; se trouve en particulier situé à la sortie de l'extrudeur ;
- des moyens de chauffage et de refroidissement car le fourreau doit être régulé en température de 60 à 300°C ;
- des moyens de pilotage de l'extrudeur tels que : un groupe d'entrainement composé d'un moteur réducteur et d'un diviseur de couple, qui fournissent la puissance mécanique nécessaire à la rotation des vis ; automates de pilotage qui permettent le suivi et la commande du procédé. Les paramètres pouvant être réglés sont : la vitesse de rotation des vis et la température de chaque fourreau.

Dans un mode de réalisation particulier, l'extrudeur est un extrudeur bi-vis-à-vis corotatives et copénétrantes.

Dans certains modes de réalisation, le procédé met en œuvre un extrudeur et de préférence un extrudeur bi-vis, à plusieurs fourreaux et terminé par un fourreau filtrant, permettant de faire varier la température et en même temps d'appliquer un cisaillement, un malaxage intense de la matière première végétale, avec pour conséquence d'entrainer un grand nombre de composés, de déstructurer la matière mais aussi d'inhiber en même temps les enzymes endogènes par un traitement thermique.

Le procédé de préparation d'un jus d'avoine fraiche consiste donc à extruder des plantes fraiches ou congelées d'avoine, en particulier les parties aériennes de ces plantes, et plus particulièrement les parties aériennes de plantules d'avoine fraiches, voire congelées. Ceci afin d'en extraire un jus, puis à procéder à la récupération et la purification (collecte) de ce jus et enfin dans une dernière étape optionnelle, stabiliser le jus ainsi collecté.

Selon une variante, le jus récupéré peut être soumis à une étape ultérieure de stabilisation, clarification et/ou filtration.

Le jus d'avoine fraiche, particulièrement le jus des parties aériennes d'avoine fraiche ou de plantules d'avoine fraiche, utile dans le cadre de l'invention, est obtenu par un traitement thermomécanique comprenant ou consistant en une opération de trituration des plantes fraiches, en particulier desdites parties aériennes, par cisaillement à des températures comprises entre 60°C et 300°C, de préférence entre 60°C et 150°C, plus particulièrement entre 75°C et 140°C, plus particulièrement encore entre 80°C et 130°C, et encore plus particulièrement aux environs de 120°C.

Avantageusement, le traitement thermomécanique est mis en œuvre dans un extrudeur bi-vis comportant une première zone bi-vis corotatives et copénétrantes où se réalise la trituration des plantes, parties de plantes, plantules ou parties de plantules, et une seconde zone bi-vis séparées où se réalise la séparation solide/liquide. L'écoulement dans la zone bi-vis est généré par un effet de pompage et non par des forces de frottements entre vis et fourreau comme cela apparait dans un extrudeur mono-vis.

Avantageusement, l'alimentation, le transport, le cisaillement mécanique et le traitement thermomécanique permettant la trituration des plantes fraiches et l'extraction du jus sont réalisés dans la première zone de l'extrudeur, et l'opération de séparation liquide/solide est réalisée dans la seconde zone.

De manière avantageuse, la première zone comporte plusieurs fourreaux successifs dont les températures sont réglées de façon à présenter des étapes de températures croissantes échelonnées entre 60°C et 130°C, et la deuxième zone comporte au moins un fourreau porté à une température comprise entre 30°C et 130°C, de préférence entre 30°C et 100°C.

Ainsi, les plantes fraiches, en particulier les parties aériennes d'avoine fraiche, ou les plantules d'avoine fraiches, en particulier les parties aériennes de plantules d'avoine fraiches sont extraites par un procédé mécanique d'extrusion en utilisant un extrudeur bi-vis, aux environs de 100 à 130°C, en particulier aux environs de 120°C. C'est à cette température que l'extrusion des flavonoïdes de la matière végétale est optimale. Le jus ainsi obtenu peut être filtré et subir une ultrafiltration, par exemple avec un seuil de coupure de 3,5kDa. En plus de l'inactivation des protéines dans l'extrudeur, cette filtration permet de se débarrasser des dernières traces de protéines. Enfin, le jus peut subir une filtration stérilisante et stabilisation en présence de glycérine végétale. L'extrusion des plantes fraiche d'avoine, particulièrement des parties aériennes de plantules d'avoine fraiche, permet d'obtenir :

- un bon rendement en jus,
- un bon rendement en matière sèche extraite,
- un bon rendement en flavonoïdes,
- la présence dans le jus des saponines non hydrolysées.

L'analyse d'un jus d'avoine fraiche obtenu par un tel procédé donne ainsi :

- des flavonoïdes : isovitexine-2''-O-arabinoside, isoorientine-2''-O-arabinoside, teneur : 2 à 4%, particulièrement environ 2,5% en poids par rapport à la matière sèche (0,1 à 1, particulièrement environ 0,2% en poids par rapport au jus pur),
- des sucres totaux : glucose, galactose, fructose, teneur de 40 à 50% en poids par rapport à la matière sèche, particulièrement environ 44%,
- des minéraux : Mg (0,3 à 0,4% en poids, particulièrement environ 0,6% par rapport à la matière sèche), Ca (0,1 à 0,5% en poids, particulièrement 27%, par rapport à la matière sèche),
- des protéines : pas de protéines détectées dans le perméat (<1,2ppm par électrophorèse),
- un taux de matière sèche compris entre 2 et 20% de matière sèche, particulièrement entre 4 et 18%, plus particulièrement encore entre 5 et 15% de matière sèche en poids, par rapport au poids du jus.

L'association de la présente invention comprend typiquement un ratio massique extrait sec de Garcinia mangostana L./jus d'*Avena sativa* fraiche allant de 0,0002/1 à 5000/1, de préférence allant de 0,005/1 à 1/1 ou de 0,01/1 à 0,5/1 ou de 0,04/1 à 0,16/1.

COMPOSITIONS

La présente invention concerne aussi une composition cosmétique ou dermatologique comprenant une association selon l'invention, c'est-à-dire comprenant un extrait de Garcinia mangostana, un jus d'*Avena sativa* fraiche, et au moins un excipient cosmétiquement ou dermatologiquement acceptable.

L'extrait de Garcinia mangostana et le jus d'*Avena sativa* fraiche peuvent être tels que décrits ci-dessus. Dans la présente invention, on entend désigner par « cosmétiquement ou dermatologiquement acceptable », ce qui est utile dans la préparation d'une composition cosmétique, dermatologique, qui est généralement sûr, non toxique et ni biologiquement ni autrement non souhaitable et qui est acceptable pour une utilisation cosmétique ou dermatologique, notamment par application topique sur la peau, notamment au niveau du visage.

Dans certains modes de réalisation, l'extrait de Garcinia mangostana et le jus d'*Avena sativa* fraiche sont les seuls principes actifs de la composition cosmétique ou dermatologique.

L'invention vise de préférence des compositions cosmétiques ou dermatologique se présentant sous une forme adaptée à une application topique.

Par « application topique », on entend une application sur la peau, les muqueuses, notamment au niveau du visage.

Les compositions cosmétique ou dermatologique peuvent se présenter ainsi sous les formes qui sont habituellement connues pour une administration topique, c'est-à-dire notamment les lotions, les shampoings, les baumes, les mousses, les gels, les dispersions, les émulsions, les sprays, les sérums, les masques, les crèmes ou les sticks avec des excipients permettant notamment, pour certains, une pénétration afin d'améliorer les propriétés et l'accessibilité des principes actifs.

La composition cosmétique ou dermatologique, outre l'extrait de Garcinia mangostana et le jus d'*Avena sativa* fraiche et un excipient cosmétiquement acceptable, peut également contenir des agents tensioactifs, des agents complexants, des conservateurs, des antioxydants (comme les tocophérols), des agents stabilisants, des émulsifiants, des épaississants, des gélifiants, des humectants, des émollients, des oligo-éléments, des huiles essentielles, des parfums, des colorants, des agents matifiants, des filtres chimiques ou minéraux, des agents hydratants, des eaux thermales, etc.

Dans certains modes de réalisation, la composition cosmétique ou dermatologique comprend un extrait de péricarpe du fruit de Garcinia mangostana.

Dans certains modes de réalisation, la composition cosmétique ou dermatologique comprend un extrait hydro-alcoolique de Garcinia mangostana, de préférence un extrait hydro-alcoolique de péricarpe du fruit de Garcinia mangostana.

Dans certains modes de réalisation, la composition cosmétique ou dermatologique comprend un jus d'*Avena sativa* fraiche, en particulier un jus de parties aériennes d'avoine, tout particulièrement un jus de parties aériennes de plantules d'avoine. De manière préférentielle, ce jus est obtenu par un traitement thermomécanique comprenant ou consistant en une opération de trituration desdites plantes fraiches, en particulier les parties aériennes, par cisaillement à des températures comprises entre 60°C et 300°C, de préférence entre 60°C et 150°C, plus particulièrement entre 75°C et 140°C, plus particulièrement encore entre 80°C et 130°C et encore plus particulièrement aux environs de 120°C.

La composition cosmétique ou dermatologique comprend avantageusement un jus d'*Avena sativa* fraiche, particulièrement un jus de parties aériennes d'avoine ou un jus de parties aériennes de plantules d'avoine, qui comprend un taux de matière sèche compris entre 2 et 20% en poids de matière sèche, particulièrement entre 4 et 18% en poids, plus particulièrement encore entre 5 et 15% en poids de matière sèche, par rapport au poids du jus.

La composition cosmétique ou dermatologique comprend avantageusement un extrait hydro-alcoolique de Garcinia mangostana, de préférence un extrait hydro-alcoolique de péricarpe du fruit de Garcinia mangostana et un jus de parties aériennes d'*Avena sativa* fraiche, en particulier un jus de parties aériennes de plantules d'avoine. La composition cosmétique ou dermatologique comprend notamment de 0,001 à 5% en poids d'un extrait sec de Garcinia mangostana, de préférence de 0,002 à 2% en poids, de manière préférée de 0,005 à 1% en poids, de manière encore préférée de 0,01 à 0,5% en poids d'un extrait sec de Garcinia mangostana par rapport au poids total de la composition.

La composition cosmétique ou dermatologique comprend notamment d'environ 0,01 à 10% en poids de jus d'Avena sativa fraiche par rapport au poids total de la composition, en particulier d'environ 0,1 à 5% en poids, en particulier d'environ 0,2 à 5% en poids, plus particulièrement de 0,3 à 4% en poids, encore plus particulièrement de 0,4 à 3% en poids, et de façon encore plus particulière de 0,5 à 2% en poids de jus d'Avena sativa fraiche par rapport au poids total de la composition, voire environ 1% en poids de jus d'Avena sativa fraiche par rapport au poids total de la composition.

La composition cosmétique ou dermatologique comprend notamment :

- de 0,001 à 5% en poids d'un extrait sec de Garcinia mangostana, de préférence de 0,002 à 2% en poids, de manière plus préférée de 0,005 à 1% en poids, de manière encore plus préférée de 0,01 à 0,5% en poids d'extrait sec de Garcinia mangostana par rapport au poids total de la composition, et
- d'environ 0,01 à 10% en poids de jus d'Avena sativa fraiche, en particulier d'environ 0,1 à 5% en poids, en particulier d'environ 0,2 à 5% en poids, plus particulièrement de 0,3 à 4% en poids, encore plus particulièrement de 0,4 à 3% en poids, et de façon encore plus particulière de 0,5 à 2% voire environ 1% en poids de jus d'Avena sativa fraiche, par rapport au poids total de la composition.

De préférence, le jus d'Avena sativa fraiche est présent dans la composition cosmétique ou dermatologique à une teneur d'environ 0,5% en poids par rapport au poids total de la composition.

De façon également préférée, le jus d'Avena sativa fraiche est présent dans la composition cosmétique ou dermatologique selon l'invention à une teneur d'environ 1% en poids par rapport au poids total de la composition.

UTILISATIONS ET METHODES

L'invention concerne également une association comprenant un extrait de Garcinia mangostana et un jus d'*Avena sativa* fraiche ou une composition cosmétique ou dermatologique comprenant une telle association et au moins un excipient cosmétiquement ou dermatologiquement acceptable, pour son utilisation dans le traitement de l'inflammation induite par *C. acnes.*

L'extrait de Garcinia mangostana, le jus d'*Avena sativa* fraiche et la composition cosmétique ou dermatologique comprenant une telle association peuvent être tels que décrits ci-dessus.

L'invention concerne également l'utilisation d'une association comprenant un extrait de Garcinia mangostana et un jus d'*Avena sativa* fraiche pour la préparation d'une composition cosmétique ou dermatologique destinée au traitement de l'inflammation induite par *C. acnes.*

L'invention concerne également l'utilisation d'une association comprenant un extrait Garcinia mangostana et un jus d'*Avena sativa* fraiche ou d'une composition cosmétique ou dermatologique comprenant une telle association et au moins un excipient cosmétiquement ou dermatologiquement acceptable, dans le traitement de l'inflammation induite par *C. acnes.*

L'invention concerne également l'utilisation d'une composition cosmétique ou dermatologique comprenant un extrait Garcinia mangostana et un jus d'*Avena sativa* fraiche pour la préparation d'un médicament destiné au traitement de l'inflammation induite par *C. acnes.*

L'invention concerne également une méthode de traitement de l'inflammation induite par *C. acnes* comprenant l'administration à une personne en ayant besoin d'une quantité efficace d'une association comprenant un extrait Garcinia mangostana et un jus d'*Avena sativa* fraiche ou d'une composition cosmétique ou dermatologique comprenant une telle association et au moins un excipient cosmétiquement ou dermatologiquement acceptable.

L'invention concerne également une association comprenant un extrait de Garcinia mangostana et un jus d'*Avena sativa* fraiche ou une composition cosmétique ou dermatologique comprenant une telle association et au moins un excipient cosmétiquement ou dermatologiquement acceptable, pour son utilisation dans le traitement de l'acné ou des peaux acnéiques.

L'invention concerne également l'utilisation d'une association comprenant un extrait de Garcinia mangostana et un jus d'*Avena sativa* fraiche pour la préparation d'une composition cosmétique ou dermatologique destinée au traitement de l'acné ou des peaux acnéiques.

L'invention concerne également l'utilisation d'une association comprenant un extrait Garcinia mangostana et un jus d'*Avena sativa* fraiche ou d'une composition cosmétique ou dermatologique comprenant une telle association et au moins un excipient cosmétiquement ou dermatologiquement acceptable, dans le traitement de l'acné ou d'une peau acnéique.

L'invention concerne également l'utilisation d'une composition cosmétique ou dermatologique comprenant un extrait Garcinia mangostana et un jus d'*Avena sativa* fraiche pour la préparation d'un médicament destiné au traitement de l'acné ou d'une peau acnéique.

L'invention concerne également une méthode de traitement de l'acné ou d'une peau acnéique comprenant l'administration à une personne en ayant besoin d'une quantité efficace d'une association comprenant un extrait Garcinia mangostana et un jus d'*Avena sativa* fraiche ou d'une composition cosmétique ou dermatologique comprenant une telle association et au moins un excipient cosmétiquement ou dermatologiquement acceptable.

Les exemples qui suivent illustrent l'invention sans en limiter la portée.

**EXEMPLES**

Exemple 1 : Extraction à reflux à l'éthanol 90%

377 grammes de péricarpes secs broyés de Garcinia mangostana sont extraits à reflux sous agitation par 3,8 litres d'un mélange éthanol/eau 90 : 10 (v/v) pendant 1 heure dans un réacteur. L'extrait est ensuite filtré sur plaque de filtration K900 et le solvant est évaporé de manière à obtenir 100 grammes d'une poudre orangée avec un rendement massique de 26%. L'extrait sec obtenu contient 15,9% en poids d'alpha-mangostine.

Exemple 2 : Extraction à reflux à l'éthanol 90% suivie d'une mise sur support

200 grammes de péricarpes secs broyés de Garcinia mangostana sont extraits à reflux sous agitation par 2 litres d'un mélange éthanol/eau 90 : 10 (v/v) pendant 1 heure dans un réacteur. L'extrait est ensuite filtré sur plaque de filtration K900 et est ensuite séché sur maltodextrine de façon à obtenir 142 grammes d'extrait sous forme d'une poudre beige orangée. L'extrait contient 75% de maltodextrine et 3,5% d'alpha-mangostine en poids de l'extrait.

Exemple 3 : Extraction à reflux à l'éthanol 90% suivie d'une mise sur support

1000 grammes de péricarpes secs broyés de Garcinia mangostana sont extraits à reflux sous agitation par 10 litres d'un mélange éthanol/eau 90 : 10 (v/v) pendant 1 heure dans un réacteur. L'extrait est ensuite filtré sur plaque de filtration K900 et est ensuite séché sur 1,2-pentanediol de façon à obtenir 747 grammes d'extrait sous forme d'un liquide visqueux sombre. L'extrait contient 70% de 1,2-pentanediol et 4,9% d'alpha-mangostine en poids de l'extrait.

Exemple 4 : Extraction à reflux à l'hexane

17 grammes de péricarpes secs broyés de Garcinia mangostana sont extraits à reflux sous agitation par 170 millilitres d'hexane pendant 1 heure dans un réacteur. L'extrait est ensuite filtré sur plaque de filtration K900 et le solvant est évaporé de manière à obtenir 200 milligrammes d'une pâte jaune orangé avec un rendement massique de 1,2%. L'extrait sec obtenu contient 75,0% en poids d'alpha-mangostine.

Exemple 5 : Extraction à l'éthanol 96% assistée par ultra-sons

36 grammes de péricarpes secs broyés de Garcinia mangostana sont mis en contact avec 350 millilitres d'éthanol 96% puis extraits sous l'action des ultra-sons (20kHz) pendant 3 fois une minute à une amplitude de 100%. L'extrait est ensuite filtré sur plaque de filtration K900 et le solvant est évaporé de manière à obtenir 7,9 grammes d'une poudre rouge violacée avec un rendement massique de 22%. L'extrait sec obtenu contient 16,4% en poids d'alpha-mangostine.

Exemple 6 : Extraction à reflux à l'éthanol 96%

20 grammes de péricarpes secs broyés de Garcinia mangostana sont extraits à reflux sous agitation par 200 millilitres d'éthanol 96% pendant une heure dans un réacteur. L'extrait est ensuite filtré sur plaque de filtration K900 et le solvant est évaporé de manière à obtenir 5,3 grammes d'une poudre rouge violacée avec un rendement massique de 26%. L'extrait sec obtenu contient 16,0% en poids d'alpha-mangostine.

Exemple 7 : Extraction hydrotropique avec une solution aqueuse d'heptylglucoside 1,5M

26 grammes de péricarpes secs broyés de Garcinia mangostana sont extraits pendant 2 heures à 40°C sous agitation par 260 millilitres d'une solution aqueuse d'heptylglucosides 1,5M. Après filtration sur plaque de filtration K900, le filtrat est acidifié à pH=2 puis dilué avec 11 volumes d'eau acidifiée à pH=2. Après centrifugation, le culot est repris et séché de manière à obtenir une pâte orangée avec un rendement massique de 7,3%. L'extrait sec obtenu contient 17,0% en poids d'alpha-mangostine.

Exemple 8 : Extraction hydrotropique avec une solution aqueuse de butyl xylosides à 25%

20 grammes de péricarpes secs broyés de Garcinia mangostana sont extraits pendant 2 heures à température ambiante sous agitation par 200 millilitres d'une solution aqueuse de butyl xylosides à 25% massique. Après filtration sur plaque de filtration K900, le filtrat est dilué par 2 volumes d'eau. Après centrifugation, le culot est récupéré. Le surnageant est dilué par 2 volumes d'eau. Après centrifugation, le culot est de nouveau récupéré et rassemblé avec le culot précédent, de façon à obtenir l'extrait sec de Garcinia mangostana sous forme d'une poudre marron avec un rendement massique de 4,9%. L'extrait sec obtenu contient 53,0% en poids d'alpha-mangostine.

Exemple 9 : Extraction hydrotropique avec une solution aqueuse de butyl xylosides (APXC4) assistée par extrusion

670 grammes de péricarpes secs de Garcinia mangostana sont introduits dans le premier fourreau d'un extrudeur bivis Clextral BC45 avec un débit de 40 kg/h. Est ensuite introduite une solution aqueuse de butyl xylosides à 26% massique avec un débit de 120 kg/h.
La température appliquée aux différents fourreaux est de 60°C. Au bout d'une minute, l'extrait de péricarpes de Garcinia mangostana est récupéré en sortie d'extrudeur grâce à un fourreau filtrant permettant une spération solide/- liquide. Après clarification, la solution est diluée par 2 volumes d'eau. Après centrifugation, le culot est récupéré. Le surnageant est dilué par 2 volumes d'eau. Après centrifugation, le culot est de nouveau récupéré et rassemblé avec le culot précédent, de façon à obtenir l'extrait sec de Garcinia mangostana sous forme d'une pâte orangée avec un rendement massique de 0,4%. L'extrait sec obtenu contient 50,0% en poids d'alpha-mangostine.

Exemple 10 : Extraction par $CO_2$ supercritique

470 grammes de péricarpes secs broyés de Garcinia mangostana sont extraits pendant 2 heures à 50°C et sous 50 bars par du $CO_2$ supercritique dont le débit est de 10 kg/h. L'extrait est ensuite solubilisé dans de l'éthanol puis filtré sur plaque de filtration K900 et le solvant est évaporé de manière à obtenir 3,4 grammes d'une pâte jaune orangé avec un rendement massique de 0,7%. L'extrait sec obtenu contient 9,45% en poids d'alpha-mangostine.
Le marc (466 grammes) est ensuite extrait pendant 1 heure à 50°C et sous 50 bars par du $CO_2$ supercritique avec pour co-solvant de l'éthanol (débits de 10 kg/h et 1kg/h, respectivement). L'extrait est filtré sur plaque de filtration K900 et le solvant est évaporé de manière à obtenir 2,3 grammes d'une pâte jaune orangé avec un rendement massique de 0,5%. L'extrait sec obtenu contient 22,3% en poids d'alpha-mangostine.

Exemple 11 : Obtention d'un jus d'avoine fraiche par traitement thermomécanique

12,75 kg de parties aériennes fraiches décongelées (24h à 2°C) d'avoine (*Avena sativa* L.) récoltées à l'ensileuse après 2 mois de croissance (plantules d'avoine) ont été introduit dans le premier fourreau d'un extrudeur bivis à vis corotatives et copémétrantes CLEXTRAL BC45 qui en comporte 5. La température appliquée aux différents fourreaux est de 30°C / 120°C / 120°C / 120°C / 60°C.
Le schéma du procédé s'établit comme suit (durée totale de l'étape d'extrusion = 20 minutes ; débit de traitement 38 kg de plantes / h et 22 kg de jus / h) :

Parties aériennes fraiches → Extrusion 120°C → Marc extrudé
  → Jus Brut (rendement 57,2% (p/p))
    → Clarification
    → Filtration stérilisante

Jus limpide contenant 11% de matière sèche (MS)

(rendement 53,1% (p/p))

→5,8% MS / plante fraiche

Après extrusion, on obtient 57,2% de jus p/p par rapport à la matière engagée. Des étapes de clarification et de filtration stérilisante ont ensuite été réalisées afin d'obtenir un jus limpide, avec un rendement final en jus de 53,1% contenant 11% en poids de matière sèche, soit un rendement en matière sèche extraite de 5,8% (p/p)).
Le rendement en jus obtenu par pressage (broyage-pressage-filtration) de la même matière première est de 50%, contenant 4,5% de matière sèche en poids, soit un rendement de 2,25% (p/p).
La technologie d'extrusion permet donc d'obtenir plus de jus, et un jus plus riche en composés, et notamment en composés bioactifs. En effet, la teneur, en % en poids, en flavonoïdes du jus obtenu par cet exemple est de 0,26%, alors qu'il n'est que de 0,02% dans le jus obtenu par pressage avec la même matière première. La teneur en flavonoïdes a donc été multipliée par 10 dans ce cas. Ces résultats sont présentés dans le Tableau 1.
Il peut également être remarqué l'intérêt d'extruder à chaud pour la teneur en flavonoïdes : la température permet d'extraire plus de composés (dont 4 fois plus de flavonoïdes) et d'obtenir les molécules natives, non dénaturées par les enzymes.
C'est également ce qu'on remarque avec les saponines de l'avoine, les avénacosides, qui sont rapidement déglycosylées par pressage. Les molécules natives sont retrouvées seulement par traitement thermomécanique : les jus obtenus par extrusion à 120°C et 200°C contiennent bien des avénacosides (A et B) à hauteur de 89 mg et 93 mg pour 100 g de matière sèche (ES).

Ils n'ont pas été dégradés par les déglucosidases endogènes.

Tableau 1

| Technique | Paramètres | Rdt Jus * | MS (% en Poids) | Rdt MS/pl | % Flavonoïdes (% en poids) | | | Avénacosides |
|---|---|---|---|---|---|---|---|---|
| | | | | | MS | /jus | /Matière fraiche | |
| | | | | % | | | | |
| Pressage | Broyage, (1) | 51 | 3,78 | 1,94 | 0,44 | 0,02 | 0,01 | Desgluco avenacosides |
| Extrusion | 25°C | 59,70 | 7,50 | 4,47 | 0,80 | 0,06 | 0,04 | 0% |
| | 1200C | 53,13 | 11 | 5,84 | 2,40 | 0,26 | 0,15 | 89 mg %g ES |
| | 200°C | 48,07 | 10 | 4,81 | 2,30 | 0,22 | 0,12 | 93 mg %g ES |
| Extraction $H_2O$ | 1H reflux | | | 3,10 | 1,10 | | 0,03 | |
| * : après filtration ; (1) : Broyage, pressoir viticole et filtration | | | | | | | | |

Exemple 12 : Propriétés biologiques de l'association selon l'invention

Le but de cette étude est d'évaluer les propriétés anti-inflammatoires d'un extrait de Garcinia mangostana et d'un jus d'Avena sativa fraiche sur l'inflammation induite par *C. acnes.*

Pour adresser cette hypothèse, des concentrations d'extrait de Garcinia mangostana, de jus d'Avena sativa fraiche et de leur association sont évaluées sur la cascade immuno-inflammatoire induite en réponse à *C. acnes* en culture planctonique en amont des voies TH1 et Th17, sur la production de cytokines pro-inflammatoires par des cellules dendritiques dérivées à partir de monocytes.

Méthode :

Les expériences sont menées sur des cocultures de cellules dendritiques immatures dérivées de monocytes, à la concentration finale de $1.10^5$ cellules/ml/puit de *C. acnes* planctoniques.

Les monocytes sont purifiés à partir de sang humain par sélection négative (The Easy Step™ Human Monocyte Enrichment Kit, StemCell).

Au Jour 0, les monocytes sont incubés dans un milieu de différenciation (Gibco Roswell Park Memorial Institute (RPMI), 10% v/v Sérum de Veau Fœtal (SVF) décomplémenté (chauffé à 56°C pendant 30 min), 50 ng/ml IL-4 et 100 ng/ml Recombinant Human Granulocyte Macrophage Colony-Stimulating Factor (GM-CSF)). Au 3ème jour de culture, la moitié du milieu est remplacé par du milieu frais. Au jour 6, les cellules dendritiques immatures sont caractérisées par cytométrie de flux et stimulés par une suspension de *C. acnes.* Cette culture est préparée par Fonderephar (Toulouse, France) dans un milieu de culture adapté, gélose Columbia + 5% de sang de mouton (COS). Une préparation de 20 ml de suspension de C. *acnes* est faite extemporanément en bouillon BHI à environ 54% de transmission à 640 nm.

Le dénombrement est réalisé par dilution au 1/10, dans le même bouillon, jusqu'à la dilution $10^{-6}$.

Etalement de 2 x 100μl des dilutions $10^{-5}$ et $10^{-6}$, sur gélose COS. Incubation pendant 72h sous anaérobiose à 36°C.

La stimulation des cellules dendritiques dérivées des monocytes par la culture planctonique de *C. acnes* est réalisée au facteur de multiplicité d'infection (MOI) proche de 100 ;

$$MOI = [C.\ acnes] / [\text{cellules dendritiques dérivées des monocytes}].$$

L'extrait de Garcinia mangostana utilisé dans cette étude est préparé selon l'exemple 1.

Il est dilué dans du DMSO et est testé à 1,25 ; 2,5 et 10 μg/ml.

Le jus d'Avena sativa fraiche est préparé selon l'exemple 11, il est dilué dans le milieu de culture, il est testé à 10, 20 et 70 µg/ml.

Un contrôle positif pour inhiber la production des cytokines inflammatoires est également testé dans cette étude, il s'agit de la Dexaméthasone à la concentration de 300 ng/ml, solubilisée dans de l'eau.

Les surnageants sont récupérés après 24 heures de stimulation.

Les résultats sont moyennés à partir de 3 expériences indépendantes.

Deux cytokines sont évaluées dans cette étude : IL-6 et IL-12p40, ces cytokines sont quantifiées en multiplex via la technologie Luninex (Bioplex 200, Biorad).

Résultats :

Les effets d'un extrait de Garcinia mangostana et de jus d'Avena sativa fraiche, seul ou en combinaison sur la production d'interleukines 6 (IL-6) par des cellules dendritiques dérivées des monocytes, stimulées avec les cultures planctoniques de C. *acnes* sont présentés dans le tableau 2 ci-dessous.

Tableau 2

| Produits | Concentration | Production moyenne d'IL-6 (%) | % d'inhibition | Statistiques |
|---|---|---|---|---|
| Pas de stimulation | - | 0 | - | |
| Stimulation | Culture C. *acnes* | 100 | 0 | |
| Dexaméthasone | 300 ng/ml | 52,6 | 45,9 | P<0,001 |
| Extrait Garcinia mangostana (G. m.) | 1,25 µg/ml | 84,1 | | NS |
| | 2,5 µg/ml | 67,8 | 32,2 | P<0,01 |
| | 10 µg/ml | 34,7 | 62,3 | P<0,001 |
| Jus d'Avena sativa fraiche | 10 µg/ml | 76,7 | | NS |
| | 20 µg/ml | 84,3 | | NS |
| | 70 µg/ml | 90,6 | | NS |
| Extrait G. m + Jus d'Avena sativa fraiche | 1,25 µg/ml+10 µg/ml | 71,3 | 28,7 | P<0,05 |
| | 2,5 µg/ml + 20 µg/ml | 61,4 | 38 ,6 | P<0,001 |
| | 10 µg/ml + 70 µg/ml | 34,2 | 63,3 | P<0,001 |
| NS : non significatif. | | | | |

Sans stimulation, les cellules dendritiques dérivées des monocytes ne produisent pas d'IL-6. Cependant, la culture planctonique de C. *acnes* induit significativement la production d'IL-6 par les cellules dendritiques dérivées des monocytes. Le contrôle positif, la dexaméthasone à 300 ng/ml, inhibe de façon significative cette production, ces résultats attendus permettent de valider ce test.

L'extrait de Garcinia mangostana à 1,25 µg/ml ne modifie pas la libération d'IL-6 par rapport à la condition de stimulation par la culture de C. *acnes,* en revanche, à partir de 2,5 µg/ml l'extrait de Garcinia mangostana inhibe significativement la production d'IL-6 par les cellules dendritiques dérivées des monocytes. Le jus d'Avena sativa fraiche à toutes les concentrations testées, est dénué d'effets sur la production d'IL-6 par les cellules dendritiques dérivées des monocytes. En revanche, l'association d'un extrait de Garcinia mangostana à 1,25 µg/ml et de jus d'Avena sativa fraiche à 10 µg/ml inhibe de manière significative la production d'IL-6 avec un effet synergique. De même aux concentrations intermédiaires, l'association d'un extrait de Garcinia mangostana et de jus d'Avena sativa fraiche permet de démasquer un effet inhibiteur statistiquement plus important (P<0,001) versus les agents utilisés individuellement.

Les effets d'un extrait de Garcinia mangostana et de jus d'Avena sativa fraiche, seul ou en combinaison sur la production d'interleukines 12p40 (IL-12p40) par des cellules dendritiques dérivées des monocytes, stimulées avec les cultures planctoniques de C. *acnes* sont présentés dans le tableau 3 ci-dessous.

Tableau 3

| Produits | Concentration | Production moyenne d'IL-12p40 (%) | % d'inhibition | Statistiques |
|---|---|---|---|---|
| Pas de stimulation | - | 0 | - | |

(suite)

| Produits | Concentration | Production moyenne d'IL-12p40 (%) | % d'inhibition | Statistiques |
|---|---|---|---|---|
| Stimulation | Culture *C. acnes* | 100 | 0 | |
| Dexaméthasone | 300 ng/ml | 49,4 | 50,0 | P<0,001 |
| Extrait Garcinia mangostana (G. m.) | 1,25 $\mu$g/ml | 89,9 | | NS |
| | 2,5 $\mu$g/ml | 78,6 | 21,4 | P<0,05 |
| | 10 $\mu$g/ml | 57,2 | 41,8 | P<0,001 |
| Jus d'Avena sativa fraiche | 10 $\mu$g/ml | 78,4 | | NS |
| | 20 $\mu$g/ml | 87,1 | | NS |
| | 70 $\mu$g/ml | 87,4 | | NS |
| Extrait G. m + Jus d'Avena sativa fraiche | 1,25 $\mu$g/ml+10 $\mu$g/ml | 84,7 | | NS |
| | 2,5 $\mu$g/ml + 20 $\mu$g/ml | 71,9 | 28,1 | P<0,01 |
| | 10 $\mu$g/ml + 70 $\mu$g/ml | 50,6 | 48,4 | P<0,001 |
| NS : non significatif. | | | | |

Sans stimulation, les cellules dendritiques dérivées des monocytes ne produisent pas d'IL-12p40. Cependant, la culture planctonique de *C. acnes* induit significativement la production d'IL-12p40 par les cellules dendritiques dérivées des monocytes. Le contrôle positif, la dexaméthasone à 300 ng/ml, inhibe de façon significative cette production, ces résultats attendus permettent de valider ce test.

L'extrait de Garcinia mangostana à 1,25 $\mu$g/ml ne modifie pas la libération d'IL-12p40 par rapport à la condition de stimulation par la culture de *C. acnes,* en revanche, à 2,5 et 10 $\mu$g/ml l'extrait de Garcinia mangostana inhibe significativement la production d'IL-12p40 par les cellules dendritiques dérivées des monocytes de 21,4 et 41,8 %, respectivement. Le jus d'Avena sativa fraiche à toutes les concentrations testées, est dénué d'effets sur la production d'IL-6 par les cellules dendritiques dérivées des monocytes. En association, l'extrait de Garcinia mangostana à 2,5 $\mu$g/ml et le jus d'Avena sativa fraiche à 20$\mu$g/ml inhibe significativement (28,1%) la production d'IL-12p40 par les cellules dendritiques dérivées des monocytes avec un effet synergique. De la même façon, l'extrait de Garcinia mangostana à 10 $\mu$g/ml et le jus d'Avena sativa fraiche à 70$\mu$g/ml inhibe significativement (48,4%) la production d'IL-12p40 par les cellules dendritiques dérivées des monocytes avec un effet synergique.

En conclusion, les inventeurs mettent en évidence que le jus d'Avena sativa fraiche pris isolément est dénué d'effet anti-inflammatoire, et qu'un extrait de Garcinia mangostana seul présente une activité antiinflammatoire relativement modeste dans ce modèle en inhibant les productions de deux interleukines. En revanche, les inventeurs démontrent une synergie d'action pour l'activité antiinflammatoire, en associant ces deux agents.

**Revendications**

1. Association comprenant un extrait de Garcinia mangostana et un jus d'Avena sativa fraiche.

2. Association selon la revendication 1, **caractérisée en ce que** l'extrait de Garcinia mangostana est un extrait de péricarpe du fruit de Garcinia mangostana.

3. Association selon la revendication 1 ou 2, **caractérisée en ce que** l'extrait de Garcinia mangostana est un extrait hydro-alcoolique.

4. Association selon l'une quelconque des revendications 1 à 3, **caractérisée en ce que** le jus d'*Avena sativa* fraiche est un jus de parties aériennes d'avoine, en particulier un jus de parties aériennes de plantules d'avoine.

5. Association selon l'une quelconque des revendications 1 à 4, **caractérisé en ce que** le jus d'*Avena sativa* fraiche, particulièrement le jus des parties aériennes d'avoine fraiche ou de plantules d'avoine fraiche, est obtenu par un traitement thermomécanique comprenant ou consistant en une opération de trituration des plantes fraiches, en particulier desdites parties aériennes, par cisaillement à des températures comprises entre 60°C et 300°C, de préférence entre 60°C et 150 0C, plus particulièrement entre 75°C et 140°C, plus particulièrement encore entre 80°C et 130°C et encore plus particulièrement aux environs de 120°C.

6. Association selon l'une quelconque des revendications 1 à 5, **caractérisé en ce que** le jus d'*Avena sativa* fraiche, particulièrement le jus des parties aériennes d'avoine fraiche ou de plantules d'avoine fraiche, comprend un taux de matière sèche compris entre 2 et 20% de matière sèche, particulièrement entre 4 et 18%, plus particulièrement encore entre 5 et 15% de matière sèche en poids, par rapport au poids du jus.

7. Association selon l'une quelconque des revendications 1 à 6, pour son utilisation dans le traitement de l'inflammation induite par *C. acnes.*

8. Association selon l'une quelconque des revendications 1 à 6, pour son utilisation dans le traitement d'une peau acnéique.

9. Composition cosmétique ou dermatologique comprenant une association selon l'une quelconque des revendications 1 à 6 avec au moins un excipient cosmétiquement ou dermatologiquement acceptable.

10. Composition selon la revendication 9, **caractérisée en ce qu'**elle comprend de 0,001% à 5%, de préférence de 0,005 à 1% d'extrait de Garcinia mangostana en poids d'extrait sec par rapport au poids total de la composition.

11. Composition selon la revendication 9 ou 10, **caractérisée en ce que** le jus d'*Avena sativa* fraiche, particulièrement le jus des parties aériennes d'avoine fraiche ou de plantules d'avoine fraiche, est présent dans la composition cosmétique ou dermatologique à une teneur d'environ 0,1 à 10% en poids, particulièrement d'environ 0,2 à 5% en poids, plus particulièrement encore de 0,5 à 2% en poids, voire encore environ 1% en poids, par rapport au poids total de la composition.

12. Composition selon l'une quelconque des revendications 9 à 11, **caractérisée en ce qu'**elle se présente sous une forme adaptée à une application topique.

13. Composition selon l'une quelconque des revendications 9 à 12, pour son utilisation dans le traitement de l'inflammation induite par *C. acnes.*

14. Composition selon l'une quelconque des revendications 9 à 13, pour son utilisation dans le traitement d'une peau acnéique.


**Patentansprüche**

1. Kombination, umfassend einen Extrakt aus Garcinia mangostana und einen Saft aus frischem Avena sativa.

2. Kombination nach Anspruch 1, **dadurch gekennzeichnet, dass** der Extrakt aus Garcinia mangostana ein Extrakt aus der Fruchtwand der Garcinia mangostana-Frucht ist.

3. Kombination nach Anspruch 1 oder 2, **dadurch gekennzeichnet, dass** der Extrakt aus Garcinia mangostana ein hydroalkoholischer Extrakt ist.

4. Kombination nach einem der Ansprüche 1 bis 3, **dadurch gekennzeichnet, dass** der Saft aus frischem *Avena sativa* ein Saft aus den oberirdischen Teilen von Hafer ist, insbesondere ein Saft aus den oberirdischen Teilen von Haferkeimlingen.

5. Kombination nach einem der Ansprüche 1 bis 4, **dadurch gekennzeichnet, dass** der Saft aus frischem *Avena sativa,* insbesondere der Saft aus den oberirdischen Teilen von frischem Hafer oder von frischen Haferkeimlingen, durch eine thermomechanische Behandlung gewonnen wird, die einen Zerkleinerungsvorgang der frischen Pflanzen, insbesondere der oberirdischen Teile, durch Scheren bei Temperaturen zwischen 60°C und 300°C, vorzugsweise zwischen 60°C und 150°C, vor allem zwischen 75°C und 140°C, vor allem weiterhin zwischen 80°C und 130°C und weiterhin vor allem bei etwa 120°C umfasst oder daraus besteht.

**6.** Kombination nach einem der Ansprüche 1 bis 5, **dadurch gekennzeichnet, dass** der Saft aus frischem *Avena sativa,* insbesondere der Saft aus den oberirdischen Teilen von frischem Hafer oder von frischen Haferkeimlingen, einen Trockensubstanzgehalt zwischen 2 und 20 Gew.-% Trockensubstanz, insbesondere zwischen 4 und 18 Gew.-%, vor allem weiterhin zwischen 5 und 15 Gew.-% Trockensubstanz, bezogen auf das Gewicht des Saftes, umfasst.

**7.** Kombination nach einem der Ansprüche 1 bis 6 zu ihrer Verwendung bei der Behandlung von durch *C. acnes* verursachten Entzündungen.

**8.** Kombination nach einem der Ansprüche 1 bis 6 zu ihrer Verwendung bei der Behandlung von Akne-Haut.

**9.** Kosmetische oder dermatologische Zusammensetzung, die eine Kombination nach einem der Ansprüche 1 bis 6 mit mindestens einem kosmetisch oder dermatologisch akzeptablen Trägerstoff umfasst.

**10.** Zusammensetzung nach Anspruch 9, **dadurch gekennzeichnet, dass** sie 0,001 bis 5 Gew.-%, vorzugsweise 0,005 bis 1 Gew.-% Extrakt aus Garcinia mangostana Trockenextrakt, bezogen auf das Gesamtgewicht der Zusammensetzung, umfasst.

**11.** Zusammensetzung nach Anspruch 9 oder 10, **dadurch gekennzeichnet, dass** der Saft aus frischem *Avena sativa,* insbesondere der Saft aus den oberirdischen Teilen von frischem Hafer oder von frischen Haferkeimlingen, in der kosmetischen oder dermatologischen Zusammensetzung in einem Gehalt von etwa 0,1 bis 10 Gew.-%, insbesondere von etwa 0,2 bis 5 Gew.-%, vor allem weiterhin von 0,5 bis 2 Gew.-%, ja sogar von etwa 1 Gew.-%, bezogen auf das Gesamtgewicht der Zusammensetzung, vorliegt.

**12.** Zusammensetzung nach einem der Ansprüche 9 bis 11, **dadurch gekennzeichnet, dass** sie in einer für eine topische Anwendung geeigneten Form vorliegt.

**13.** Zusammensetzung nach einem der Ansprüche 9 bis 12 zu ihrer Verwendung bei der Behandlung von durch C. *acnes* verursachten Entzündungen.

**14.** Zusammensetzung nach einem der Ansprüche 9 bis 13 zu ihrer Verwendung bei der Behandlung von Akne-Haut.

**Claims**

**1.** An association comprising an extract of Garcinia mangostana and fresh Avena sativa juice.

**2.** The association according to claim 1, **characterized in that** the Garcinia mangostana extract is a pericarp extract of the Garcinia mangostana fruit.

**3.** The association according to claim 1 or 2, **characterized in that** the Garcinia mangostana extract is a hydro-alcoholic extract.

**4.** The association according to any one of claims 1 to 3, **characterized in that** the fresh *Avena sativa* juice is an oat aerial parts juice, in particular an oat seedling aerial parts juice.

**5.** The association according to any one of claims 1 to 4, **characterized in that** the fresh *Avena sativa* juice, particularly the juice from the aerial parts of fresh oats or of fresh oat seedlings, is obtained by a thermomechanical treatment comprising or consisting of a grinding operation of the fresh plants, in particular of said aerial parts, by shearing at temperatures comprised between 60°C and 300°C, preferably between 60°C and 150°C, more particularly between 75°C and 140°C, more particularly still between 80°C and 130°C and even more particularly around 120°C.

**6.** The association according to any one of claims 1 to 5, **characterized in that** the fresh *Avena sativa* juice, particularly the juice from the aerial parts of fresh oats or of fresh oat seedlings, comprises a dry matter content comprised between 2 and 20% dry matter, particularly between 4 and 18%, more particularly between 5 and 15% dry matter by weight, relative to the weight of the juice.

**7.** The association according to any one of claims 1 to 6, for use in the treatment of inflammation induced by *C. acnes.*

8. The association according to any one of claims 1 to 6, for use in the treatment of acne-prone skin.

9. A cosmetic or dermatological composition comprising an association according to any one of claims 1 to 6 with at least one cosmetically or dermatologically acceptable excipient.

10. The composition according to claim 9, **characterized in that** it comprises from 0.001% to 5%, preferably from 0.005% to 1% of Garcinia mangostana extract by weight of dry extract relative to the total weight of the composition.

11. The composition according to claim 9 or 10, **characterized in that** the fresh *Avena sativa* juice, particularly the juice of the aerial parts of fresh oats or of fresh oat seedlings, is present in the cosmetic or dermatological composition at a content of about 0.1 to 10% by weight, particularly about 0.2 to 5% by weight, more particularly 0.5 to 2% by weight, or even about 1% by weight, relative to the total weight of the composition.

12. The composition according to any one of claims 9 to 11, **characterized in that** it is in a form suitable for topical application.

13. The composition according to any one of claims 9 to 12, for use in the treatment of inflammation induced by *C. acnes*.

14. The composition according to any one of claims 9 to 13, for use in the treatment of acne-prone skin.

**EP 4 504 229 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2938439
- WO 2015040135 A

**Littérature non-brevet citée dans la description**

- **DAGNELIE et al.** *Journal of the European Academy of Dermatology*, 2019, vol. 33 (12), 2340-2348
- **OVALLE-MAGALLANES et al.** *Food Chem Toxicol*, 2017, vol. 109, 102-122
- **UDANI et al.** *Nutr J*, 2009, vol. 8, 48
- **MAHENDRA et al.** *J Investig Clin Dent*, 2017, vol. 8 (4)